# EUROPEAN PATENT SPECIFICATION

(11) **EP 0 582 686 B1**
(45) Date of publication and mention of the grant of the patent: **12.08.1998**
(21) Application number: 92917323.5
(22) Date of filing: 24.03.1992
(51) Int. Cl.: A61N 5/06, A61B 17/36, G02B 6/00

(54) **APPARATUS USING A LASER LUCENT NEEDLE**
VORRICHTUNG MIT LASERLICHTDURCHLAESSIGER NADEL
APPAREIL UTILISANT UNE AIGUILLE TRANSPARENTE AU LASER

(30) Priority: 05.04.1991 US 681225
(43) Date of publication of application: 16.02.1994
(60) Divisional application: 97104255.1
(73) Proprietor: INDIGO MEDICAL, INCORPORATED, Palo Alto, CA 94303 (US)
(72) Inventor: CONN, Richard, L., Albuquerque, NM 87107 (US); LOREE, Thomas, Robert, Santa Fe, NM 87501 (US); BIGIO, Irving, Joseph, Los Alamos, NM 87544 (US); STROBL, Karlheinz, Santa Fe, NM 87501 (US)
(74) Representative: Mercer, Christopher Paul
(86) International application number: US9202385
(87) International publication number: WO9217243

(56) References cited:
- EP-A- 292 695
- EP-A- 394 446
- EP-A- 450 149
- WO-A-94/04879
- DE-A- 2 826 383
- US-A- 4 269 192
- US-A- 4 273 109
- US-A- 4 336 809
- US-A- 4 736 743
- US-A- 4 950 267

## Description

The present invention relates to apparatus for laser-induced hyperthermia or photodynamic therapy, more particularly to an apparatus combining an optical fiber with a laser lucent needle.

As is well known, lasers produce an intense beam of electromagnetic radiation of high spectral purity which can be collimated to a fine degree with high radiation densities, small angular divergences, and long coherence length. Such properties make lasers particularly attractive for a variety of medical uses.

The use of lasers to induce hyperthermia in tissue involves the insertion of an optical fiber into tissue. Without any additional protection, the fiber must be able to withstand insertion and withdrawal forces without fracturing or shedding material. Any accident during hyperthermia which leaves behind material which may not be easily retrieved could be disastrous.

Additionally, it is desirable to control the diffusion of laser energy transmitted from the end of the optical fiber. The amount of scattering of laser energy in a particular tissue can be a critical factor in hyperthermia treatment. The degree of tissue scattering will determine the required diffusion of the energy transmitting end of the optical fiber. Therefore, it is desirable to provide a means for controlling the energy transmitted by the end of the optical fiber by diffusing the radiated energy for reduced energy density at the surface of the needle. This provides a needle energy delivery system that will prevent the development and deposit of charred materials on its surface while providing a design that will resist fracturing and the shedding of material. The reduction in energy density achieved by these techniques allows control of the thermal profile, which not only prevents charring and blocking of the photons, but also allows the optimization of hyperthermia therapy required to achieve the desired clinical results.

Alternative uses of the laser technology include the use of lasers to treat tumors, where the requirement of matching the heated volume to the size of the tumor can best be met by an apparatus in which the irradiation wavelength, and thus the penetration depth of the laser energy, can be changed. Although fluorescent dyes are useful for the conversion of the irradiation wavelength, the introduction of generally carcinogenic dyes into human tissue can cause serious health risks. Therefore, it is desirable to provide an apparatus which utilizes fluorescent dyes to convert the irradiation wavelength, while preventing the dye from leaking into the surrounding tissue.

DE-A-2826383 describes a tissue penetrating probe consisting of an open ended tube with an optical fibre coaxially mounted therein. Encasement of an optical fibre in a needle is itself known, for example, in US-A-4336809, issued to Clark, a transparent cladding is in close contact with an optical fibre core. The optical needle disclosed by Clark acts as a linear side radiator.

EP-A-0292695 discloses an apparatus for high intensity irradiation of objects, in which an optical fibre is surrounded at one end by a transparent cap or a balloon catheter.

A probe for photoepilation is disclosed in US-A-3834391, issued to Block. In that patent, a long, shallow tapered body terminates in a tapered tip, which holds the energy transmitting end of the fibre. The polished end of the fibre is flush with the exterior surface of the body. The specification of the Block patent cautions that the user must be careful in handling the probe because breakage of the sheathed fibre is possible.

A laser probe described in US-A-4950267 has a tapered tip formed of sapphire from which the laser beam is emitted. Light is emitted from the tip only.

A laser optic device for the treatment of blood vessels is disclosed in US-A-4564011, issued to Goldman. An optical fibre is concentrically disposed within a catheter, upon which a tapered probe is mounted such that the optical fibre itself is not in a penetrating portion in use. Laser energy is directed by a lens through the needle to an area approximately the size of the diameter of the needle.

US-A-4905689, issued to Stack, et al., discloses means for directing a laser beam circumferentially through windows that are concentrically disposed around the conical tip of the needle, rather than through a substantial portion of the shaft of the needle itself. In a preferred embodiment, the windows are formed from sapphires, rubies or diamonds and allow the laser beam to be directed to atherosclerotic plaque.

US-A-3467098, issued to Ayres, discloses a hand stylus connected to a remote laser source by a closed conduit but which does not encase an optical fibre, nor is the device insertable like a needle.

A catheter for irradiating tubular body cavities described in EP-A-0394446. An optical fibre is mounted within a transparent jacket. A space formed between the closed distal end of the jacket and the fibre is filled with a light scattering material.

US-A-4336809 describes a tissue photoradiation system. It includes a needle which consists of an optical fibre surrounded by a retractable metal sleeve. The sleeve provides added strength during implantation of the needle.

EP-A-0450149, filed on 29th October 1990 and published on 9th October 1991, relates to a fibre optic diffuser for use in photodynamic therapy. In one arrangement disclosed therein, the cladding and sheathing are removed from one end of an optical fibre to expose the fibre core. A sleeve encloses the core tip without touching the fibre core and is fixed on the sheathing of the fibre adjacent the core tip. The diffusing surface of the drilled out sleeve scatters the light spreading out from the polished core end of the fibre. The sleeve has a sharpened head portion to facilitate insertion into a tumor.

The present invention is directed at apparatus for the transmission of laser radiation to tissue, comprising a source of laser radiation; a hollow needle which is transparent to laser radiation and has a shaft and a closed tip; and at least one optical fibre within and spaced from the interior surface of the needle, the fibre being adapted to transmit laser radiation from one end and connected to the laser source at the other end. According to the invention, the needle is tapered at the tip such that it is capable of penetrating tissue upon interstitial insertion therein and is adapted to transmit laser radiation from a portion of the shaft extending from the tip to behind the one end of the fibre, the closed tip of the needle being formed with refractive scattering material therein which is adapted to diffuse the energy transmitted from the fibre.

The scattering material, which is preferably high-refractive-index powder or material, may be positioned within the walls as well as at the end of the needle to provide a means for diffusing the laser energy. This not only lowers the power density at the tissue interface to increase the safety of hyperthermia, but also simplifies the design and manufacturing of the optical fibre.

The present invention provides a laser lucent needle which may be used as a sheath for the insertion of optical fibres and diffusing tips, and then retrieved completely intact without a significant risk of leaving foreign material behind in the tissue. The optical fibre is positioned within a laser transparent needle which permits the transmission of laser energy through the walls of the needle. Any optical fibre fragments resulting from the fracturing or shedding of the fibre are thereby minimized or eliminated. This eliminates a significant risk of leaving foreign material behind in the tissue. Consequently, the transparent needle permits the use of inexpensive and reusable optical fibres and diffusion tips.

It is a feature of the present invention to space the needle shaft from the internally disposed optical fibre. This permits liquid or gas cooling of the fibre, the needle and the adjacent tissue and permits the introduction of scatterers, diffusers or wavelength-shifting dyes within the interior of the needle. The ability to cool the tissue adjacent to the inserted needle enables improved control over the thermal profile in the tissue volume, which can be critical in hyperthermia.

A spacer may be disposed within the needle to retain the fibre in a particular position within the needle, for example centrally.

These alternative arrangements of the transparent needle provide a means to customize the needle for differing hyperthermia and/or surgical or clinical requirements. However, the present invention consistently provides an inexpensive, disposable or reusable means to protect the optical fibre from insertion and withdrawal forces and to reduce the risk of introducing fibre fragments into the tissue. Ultimately, the transparent needle and laser lucent apparatus of the present invention make it feasible to use less expensive laser equipment in the clinical application of laser energy interstitially in living system.

The apparatus of the invention may be used for the hyperthermia treatment of benign prostate hypertrophy (BPH). While it is well known that lasers can be used to induce hyperthermia in tissue, almost all of the clinical applications for such hyperthermia have been associated with the treatment of malignant tumours. Diffusing tips have been designed by others and used for photodynamic therapy in volumes of fluids, such as inside the bladder. Current techniques for hyperthermia of the prostate involve microwaves and heated probes; diffusion of laser light during interstitial applications has not been standardized in practice. The present invention facilitates the treatment of BPH with laser-induced hyperthermia. An optical fibre is inserted into the lucent needle which has been inserted directly into the prostate through, for example, a rectal approach under ultrasonic visualization and guidance. Particularly in such an application, integrity of the components is critical. Portions of the tip must not be broken off and left embedded in the tissue. The fibre mount itself must be able to withstand insertion and withdrawal without fracturing or shredding material. An accident during hyperthermia that leaves material behind within the prostate could be disastrous since there is no easy way to retrieve this material.

Use of concepts of this invention, can assure proper and safe diffusion of the light within the prostate tissue. By inserting a transparent needle into the prostate (or tumour) as a sheath for the insertion of an optical fibre, retrieval can be complete without any significant risk of leaving foreign material behind, and with minimum side effects.

The invention will now be described by way of example and with reference to the accompanying drawings wherein:
Figure 1 is a partial sectional view of an apparatus incorporating a diffusing tip placed on the energy transmitting end of the optical fibre;
Figure 2 is a partial sectional view of an apparatus illustrating the incorporation of multiple optical fibres and a scatterer which is adapted to retain the energy transmitting ends of the fibres in position within the needle;
Figure 3 is a partial sectional view of an apparatus in accordance with the invention with refractive scattering material positioned within the walls and closed end of the needle;
Figure 4 is partial sectional view of a double needle apparatus including a liquid contained within the core of the interior needle; and
Figure 5 is a schematic view of the use of apparatus of this invention in the hyperthermia treatment of BPH.

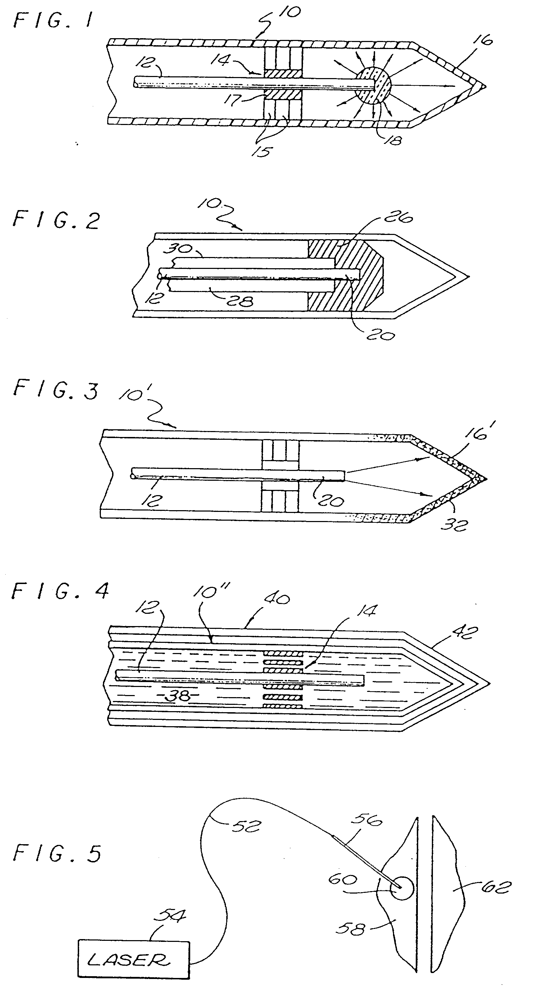

Figure 1 illustrates some of the features of the present invention, but the configuration shown falls outside the scope of the invention. It shows a laser lucent apparatus formed of a hollow, generally cylindrical, transparent needle 10, concentrically disposed about a commercially available optic fiber 12. The needle has a shaft and a tip and is preferably constructed of optical quality plastic, such as polycarbonate or polymethylmethacrylate (PMMA), so that the needle 10 may transmit laser energy at least through a substantial portion of the length of the shaft. As illustrated in FIGURE 1, energy may radiate outward from the fiber 12 and be transmitted through the tip and shaft of the needle, including a portion of the shaft located behind the tip of the fiber.

Preferably, the outside diameter of the needle 10 may be as large as 3 millimeters. However, the size of the outside diameter will be determined by the desired usage of the present invention. The needle wall is thin relative to the needle diameter, e.g., 0.3 millimeters, to minimize the heating of the wall resulting from the transmission of energy from the fiber 12. Generally, a wall thickness of 0.1 to 1.0 millimeters is useful.

The fiber 12 is connected at its distal end to a laser source (not shown), and extends into a penetrating portion of the needle 10, i.e. a portion that is intended to penetrate into the tissue to be subjected to hyperthermia and/or photodynamic therapy. The fiber 12 is retained in position in the center of the needle 10 by retaining means or a spacer 14 which bridges the distance between the outside surface of the fiber 12 to the interior surface of the needle 10. Additional spacers may be incorporated and disposed along the length of the fiber 12 as required to retain the fiber 12 in position within the needle 10. In a preferred embodiment, the spacer 14 is porous to allow the flow of gas or liquid throughout the needle 10. In the configuration shown in Figure 1, the spacer 14 has porous, flexible disks 15 of polytetrafluorethylene (Teflon) and an inner layer 17 of polycarbonate. In use, the needle 10 and fibre 12 assembly is inserted within tissue as a means to help protect the optical fibre 12 from fracturing or shedding.

As illustrated in Figure 1, a closed end 16 of the needle 10 is tapered or conically shaped. The closed end 16 prevents any fragments of the fiber from exiting the needle 10, and introducing fiber fragments onto the tissue. It also prevents body fluids, such as blood, from entering the space between the fiber 12 and the needle 10, and thus interfering surrounding tissue. The distal end of the needle is open to provide for the connection of the fiber 12 to a laser source (not shown) .

In tissues having moderate scattering, the laser lucent apparatus must deliver energy to the outside surface of the needle 10 with both an acceptably low and uniform energy density and a correct angle of incidence. To effect this, a diffusing tip 18 may be placed on the energy transmitting end 20 of the fiber 12. The incorportion of a diffusing tip 18 within the interior of the needle 10 results in an increase in the diffusion of the laser energy prior to its contact with the tissue. The increased area of the needle surface, as compared to the tip area, greatly lowers the power density at the tissue interface. This alleviates a problem of overheating at the tissue/tip interface present when a bare tip is used, while irradiating the same volume of tissue.

Figure 2 illustrates a further apparatus which is outside the scope of the invention, incorporating a scatterer which is adapted to retain the energy transmitting end 20 of a bundle of fibers 12, 28 and 30 in position within the needle 10. A retainer scatterer 26 is preferably made of optical quality plastic, such as optical epoxy loaded with about 30% by weight of refractive scattering powder, such as sapphire powder and shaped such that the energy transmitting end 20 of the fibers may be matingly received within its core. As illustrated, the retainer scatterer 26 may be an abbreviated cylinder with bores extending partially through its central axis. The diameters of the bores are slightly larger than the diameter of the fibers 12 to hold the fibers 12 in a concentric position within the retainer scatterer 26. The outside surface of the scatterer 26 is constructed to abut the interior surface of the cylindrical portion of the needle 10 and its forward face is bevelled to somewhat radially distribute the laser light energy. As illustrated, the retainer spacer 26 may replace the spacer 14 as the means for retaining the fiber 12 in position within the needle 10.

The incorporation of a bundle of fibers, including a second fiber 28 and a third fiber 30 is made possible by the diameter of the needle 10. It should be understood that the number of optical fibers incorporated may be modified according to the desired usage. The energy transmitting ends of the fibers may also take several forms, such as flat or slanted, to control the angular spread of the transmitted energy.

In the case of highly-scattering tissue, only uniform spreading of the energy is required (the angle of incidence becomes irrelevant). Figure 3 illustrates a preferred embodiment of the present invention. A flat-ended fibre 12 is mounted a spaced distance from the closed end 16^{I} of the needle 10^{I}. The distance between the transmitting end of the fiber and the closed end of the needle determines the spread of the laser beam. By placing the energy transmitting end 20 of the fiber set back from the closed end 16 of the needle 10, the angular spread of the energy transmitted is increased. The incorporation of refractive scattering material 32, such as diamond powder, within the walls and the closed end of the needle 10 serves to diffuse the energy transmitted from the fiber. The scattering material 32 may be placed in the walls by admixture prior to forming the needle.

The use of laser technology to treat tumors requires matching of the heated volume to the size of the tumor. Consequently, a laser lucent apparatus for use in the treatment of tumors must be able to meet the requirements of a changeable irradiation wavelength, and thus a changeable penetration depth of the energy.

Figure 4 illustrates an apparatus which utilizes fluorescent dye conversion to modify a laser pump wavelength. Although the apparatus of Figure 4 is not within the scope of the present invention, in a preferred embodiment of the invention a liquid is disposed within the needle of the apparatus. As illustrated in Figure 4, the optical fibre 12 is concentrically positioned within the needle 10^{II} by one or more porous spacers 14. For treating tumours, lasers in the blue to green range are preferred, such as argon ion lasers. A liquid suitable for fluorescent conversion, such as a fluorescent dye 38, is disposed within the interior of the needle 10^{II}. A fluorescent dye in the color range from orange to near-infra red is preferred, however any suitable dye may be used. Although not shown, circulating means may be added to the laser lucent apparatus to circulate the dye solution through the interior of the needle 10^{II} for cooling of the dye. An added benefit of having a cooled liquid in the needle 10^{II} is that it will provide more control over the steady-state thermal profile established in the surrounding tissue, both preventing the overheating of the needle-tissue interface and keeping the inner temperature gradient flat.

Fluorescent dyes are generally carcinogenic, thus requiring safety considerations to prevent the leaking of the dye 38 from the interior of the needle 10. To this end, a second needle 40 is illustrated in FIGURE 7. The second needle 40 is also constructed of a optical quality plastic with a configuration similar to that of the needle 10^{II}. However, the second needle 40 is configured to enclose the first needle 10^{II} within its interior.

The shape of the second needle 40 is essentially a hollow cylinder with a closed end 42. The diameter of the second needle 40 is sufficiently larger than the diameter of the needle 10^{II} to allow the second needle 40 to completely enclose the first needle 10^{II} within its interior while allowing an interstitial space between the exterior surface of the first needle 10^{II} (either by coaxial cantilever support (not shown) or by one or more spacers and the interior surface of the second needle 40. Thus, the second needle 40 provides a second barrier between the fluorescent dye 38 positioned within the interior of the first 10^{II} and the surrounding environment. Safety considerations suggest a monitoring of the interstitial space between the first needle 10^{II} and the second needle 40 for the appearance of leaking fluorescent dye.

Referring to Figure 5, a method for treating BPH is shown schematically. An optical fibre 52 receiving light from a laser 54 is placed in a needle casing 56. The fibre-needle structure 52-56 can take any of the forms previously discussed herein with respect to the accompanying Figures. The fibre-needle structure is inserted in the rectum, guided visually by means of ultrasound, as known. The tissue in a region 60 surrounding the center of the lobe 58 is heated to a temperature of about 45-52°C for a period of time of from about one to about 30 minutes. This heating induces a slow necrosis of the central tissue volume of the gland, and dead cells are eventually metabolized, thus reducing the total mass of the gland. The procedure can be repeated for the other lobe 62.

By delivering the laser light through an optical fiber inserted with its tip in the targeted tissue, the region of elevated temperature is limited to exclude the capsule of the gland or any tissue external to the gland itself. Use of a needle encasing structure of any of the preceding FIGURES serves to make this procedure safe and effective with minimum side effects.

The needle may be equipped with surface thermocouples with wires carried through the interior. Additionally, fluoroscopic tracking may be facilitated by the addition of markers made of an appropriate material which are coated on the outside of the needle 10 distal to the radiation area. Asymmetric energy deposition may be achieved by adding mirror strips to the interior wall of the needle, where they may be cooled. Finally, the addition of a light return monitor to the laser lucent apparatus of the present invention may be incorporated to detect a sharp increase in energy from the fiber, which would indicate fiber breakage.

## Claims

1. Apparatus for the transmission of laser radiation to tissue, comprising a source of laser radiation (54), a hollow needle (10) which is transparent to laser radiation and has a shaft and a closed tip (16), at least one optical fibre (12) within and spaced from the interior surface of the needle, the fibre being adapted to transmit laser radiation from one end (20) and connected to the laser source (54) at the other end,
wherein
the needle (10) is tapered at the tip (16) such that it is capable of penetrating tissue upon interstitial insertion therein and is adapted to transmit laser radiation from a portion of the shaft extending from the tip to behind the one end (20) of the fibre (12), the closed tip (16) of the needle (10) being formed with refractive scattering material (32) therein which is adapted to diffuse the energy transmitted from the fibre (12).

2. Apparatus according to Claim 1 wherein refractive scattering material (32) is positioned within the side walls of the needle (10).

3. Apparatus according to Claim 1 or Claim 2 wherein the laser source (54) is adapted to generate laser radiation of an intensity sufficient to induce necrosis in tissue (60) surrounding the needle (10).

4. Apparatus according to Claim 3 wherein the laser source (54) is adapted to generate laser radiation of an intensity sufficient to raise the temperature at the outer surface of the needle (10) to within the range 45 to 52°C.

5. Apparatus according to any preceding Claim including a spacer (14) to axially support the fibre (12) within the needle (10).

6. Apparatus according to any preceding Claim comprising a liquid (38) disposed within the needle (10).

7. Apparatus according to any preceding Claim wherein the radiation density generable at the surface of said portion of the needle shaft is substantially uniform.

## Patentansprüche

1. Vorrichtung zum Übertragen einer Laserstrahlung auf ein Gewebe, mit einer Laserstrahlenquelle (54), einer hohlen Nadel (10), die gegenüber Laserstrahlung durchlässig ist und einen Schaft sowie eine geschlossene Spitze (16) aufweist, mit wenigstens einer innerhalb der Nadel angeordneten optischen Faser (12), die einen Abstand zur Innenfläche der Nadel aufweist und die dazu dient, Laserstrahlung von einem Ende (20) aus zu übertragen, und die mit ihrem anderen Ende an die Laserquelle (54) angeschlossen ist,
dadurch gekennzeichnet, daß sich die Nadel (10) an ihrer Spitze (16) derart verjüngt, daß die bei interstitialem Einführen Gewebe durchdringen kann und derart gestaltet ist, daß sie Laserstrahlung übertragen kann von einem Schaftteil, der sich von der Spitze bis hinter das eine Ende (20) der Faser (12) erstreckt, wobei die geschlossene Spitze (16) der Nadel (10) aus einem lichtbrechenden streuenden Material (32) gebildet ist, das dazu dient, die von der Faser (12) übertragene Energie zu streuen.

2. Vorrichtung nach Anspruch 1, dadurch gekennzeichnet, daß das lichtbrechende streuende Material (32) innerhalb der Seitenwände der Nadel (10) angeordnet ist.

3. Vorrichtung nach Anspruch 1 oder 2, dadurch gekennzeichnet, daß die Laserquelle (54) dazu dient, eine Laserstrahlung einer Intensität zu erzeugen, die ausreicht, um in dem die Nadel (10) umgebenden Gewebe (60) Nekrose zu erzeugen.

4. Vorrichtung nach Anspruch 3, dadurch gekennzeichnet, daß die Laserquelle (54) dazu dient, eine Laserstrahlung von einer Intensität zu erzeugen, die ausreicht, um die Tempertur der Außenfläche der Nadel (10) auf den Bereich von 45 bis 52°C zu erwärmen.

5. Vorrichtung nach einem der vorausgehenden Ansprüche, mit einem Abstandshalter (14), der die Faser (12) innerhalb der Nadel (10) axial hält.

6. Vorrichtunæææg nach einem der vorausgegangenen Ansprüche mit einer Flüssigkeit (38) in der Nadel (10).

7. Vorrichtung nach einem der vorausgegangenen Ansprüche, dadurch gekennzeichnet, daß die an der Fläche des genannten Nadelschaftbereiches erzeugbare Strahlungsdichte im wesentlichen gleichförmig ist.

## Revendications

1. Dispositif de transmission d'un rayonnement laser sur un tissu, comportant une source de rayonnement laser (54), une aiguille creuse (10) qui est transparente à un rayonnement laser et a une tige et une pointe fermée (16), au moins une fibre optique (12) située dans la surface intérieure de l'aiguille, et espacée de celle-ci, la fibre étant adaptée pour transmettre un rayonnement laser à partir d'une première extrémité (20) et étant reliée à la source laser (54) au niveau de l'autre extrémité,
dans lequel
l'aiguille (10) a une forme conique au niveau de la pointe (16) de sorte qu'elle est capable de pénétrer dans un tissu lors d'une insertion interstitielle et est adaptée pour transmettre un rayonnement laser à partir d'une partie de la tige s'étendant de la pointe vers l'arrière de la première extrémité (20) de la fibre (12), la pointe fermée (16) de l'aiguille (10) étant constituée d'un matériau de réfraction diffusant (32) qui est adapté pour diffuser l'énergie transmise par la fibre (12).

2. Dispositif selon la revendication 1, dans lequel le matériau de réfraction diffusant (32) est positionné dans les parois latérales de l'aiguille (10).

3. Dispositif selon la revendication 1 ou 2, dans lequel la source laser (54) est adaptée pour produire un rayonnement laser ayant une intensité suffisante pour induire une nécrose du tissu (60) entourant l 'aiguille (10).

4. Dispositif selon la revendication 3, dans lequel la source laser (54) est adaptée pour produire un rayonnement laser ayant une intensité suffisante pour augmenter la température au niveau de la surface extérieure de l'aiguille (10) jusque dans la plage allant de 45 à 52°C.

5. Dispositif selon l'une quelconque des revendications précédentes, comportant un dispositif d'écartement (14) pour supporter de manière axiale la fibre (12) dans l'aiguille (10).

6. Dispositif selon l'une quelconque des revendications précédentes, comportant un liquide (38) disposé dans l'aiguille (10).

7. Dispositif selon l'une quelconque des revendications précédentes, dans lequel la densité de rayonnement pouvant être produite au niveau de la surface de ladite partie de l'arbre d'aiguille est pratiquement uniforme.
